(19) Europäisches Patentamt
European Patent Office
Office européen des brevets

(11) **EP 3 321 247 B1**

(12) **EUROPEAN PATENT SPECIFICATION**

(45) Date of publication and mention
of the grant of the patent:
**20.11.2019 Bulletin 2019/47**

(21) Application number: **16824139.6**

(22) Date of filing: **12.05.2016**

(51) Int Cl.:
*C07C 27/14* (2006.01)  *B01J 23/887* (2006.01)
*B01J 35/08* (2006.01)  *C07C 47/22* (2006.01)
*C07C 57/05* (2006.01)  *B01J 10/00* (2006.01)
*B01J 19/00* (2006.01)  *B01J 27/192* (2006.01)
*C07C 57/04* (2006.01)

(86) International application number:
**PCT/JP2016/064181**

(87) International publication number:
**WO 2017/010159 (19.01.2017 Gazette 2017/03)**

(54) **METHOD FOR PRODUCING UNSATURATED ALDEHYDE AND/OR UNSATURATED CARBOXYLIC ACID**

VERFAHREN ZUR HERSTELLUNG EINES UNGESÄTTIGTEN ALDEHYDS UND/ODER UNGESÄTTIGTER CARBONSÄURE

PROCÉDÉ DE PRODUCTION D'ALDÉHYDE INSATURÉ ET/OU D'ACIDE CARBOXYLIQUE INSATURÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.07.2015 JP 2015138375**

(43) Date of publication of application:
**16.05.2018 Bulletin 2018/20**

(73) Proprietor: **Nippon Kayaku Kabushiki Kaisha Tokyo 100-0005 (JP)**

(72) Inventors:
• **SUGIYAMA Motohiko**
Sanyoonoda-shi
**Yamaguchi 757-8686 (JP)**
• **HIRAOKA Ryota**
Sanyoonoda-shi
**Yamaguchi 757-8686 (JP)**
• **KAWAMURA, Tomoyuki**
Sanyoonoda-shi
**Yamaguchi 757-8686 (JP)**
• **YASUDA Shogo**
Sanyoonoda-shi
**Yamaguchi 757-8686 (JP)**

(74) Representative: **Gille Hrabal**
**Brucknerstrasse 20**
**40593 Düsseldorf (DE)**

(56) References cited:
WO-A1-2014/181839    WO-A1-2015/008814
WO-A1-2015/008815    JP-A- H03 176 440
JP-A- H03 200 733     JP-A- H03 215 441
JP-A- H03 294 238     JP-A- H03 294 239
JP-A- H09 202 741     JP-A- S55 113 730
JP-A- 2001 048 817    JP-A- 2003 146 920
JP-A- 2003 306 457    JP-A- 2005 162 744
US-A- 5 276 178

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a method for producing an unsaturated aldehyde and/or an unsaturated carboxylic acid by subjecting an alkene to gas-phase catalytic partial oxidation with molecular oxygen or a molecular oxygen-containing gas in the presence of a complex metal oxide catalyst, to obtain a corresponding unsaturated aldehyde and/or unsaturated carboxylic acid.

Background Art

In the case of subjecting an alkene to gas-phase catalytic partial oxidation with molecular oxygen, thereby industrially producing an unsaturated aldehyde and/or an unsaturated carboxylic acid, there are generated various problems. One of them is a problem in which as the temperature at which a complex metal oxide catalyst (hereinafter also referred to as "catalyst") is exposed becomes high, an excessive oxidation reaction is promoted, resulting in a lowering of the yield of a target product, or degradation of the catalyst is promoted, whereby a desired catalyst life cannot be achieved. This problem becomes more remarkable in the case of making a raw material concentration or a space velocity high for the purpose of enhancing the productivity. This is because the gas-phase catalytic partial oxidation of the alkene is an exothermic reaction, and as a result, a local high-temperature heat storage portion (hot spot) is generated in the catalyst layer. In response to the problem of hot spot generation, there have been made various proposals in the conventional techniques.

For example, Patent Document 1 describes a method in which plural kinds of catalysts differing in occupying volume and calcination temperature, and/or kind and/or amount of alkali metal clement from each other, are prepared and filled in a multitubular oxidation reactor in such a manner that the activity increases from the raw material gas inlet side toward the outlet side in the axial direction of the tube thereof, thereby suppressing a hot spot temperature. This method is aimed to suppress excessive heat generation by filling the catalysts with controlled activity in the inlet side into which the high-concentration raw material gas is introduced. But, in regulating the activity by the occupying volume, there would be a case where a sufficient effect is not obtained because the size of the occupying volume of the catalyst is restricted by a reaction tube diameter; or there would be a possibility that in view of the matter that the catalysts are not uniformly filled, a designed reaction field is not realized, so that a sufficient catalytic performance is not exhibited.

In addition, Patent Document 2 describes a method in which a supporting amount of the catalyst is increased from the raw material gas inlet side toward the outlet side to impose a ranking in the catalytic activity, thereby suppressing a hot spot temperature on the raw material gas inlet side, whereas on the outlet side where a highly active catalyst is filled, a gas-phase catalytic partial oxidation reaction is made to reach a conversion of the raw material required from the process standpoint. However, there is involved such a problem that in the catalyst on the raw material gas inlet side where the supporting amount is low, the life is short, whereas in the catalyst on the raw material gas outlet side, the amount of the active component is large, so that the layer of the catalytically active component becomes thick, whereby the reaction heat is accumulated within the catalyst, and the selectivity is lowered.

In addition, Patent Document 3 describes a method in which by using annular catalysts, the hot spot temperature is suppressed, thereby making it possible to cope with the reaction under high-load circumstances. But, the annular catalysis also encounter such serious problems that when filled in a multitubular oxidation reactor, it is difficult to uniformly fill the catalysts from the standpoint of properties of the shape; and that from the standpoint of properties of a molding method, the catalysts collapse or cause powdering because of low mechanical strength, so that not only the reaction tube is plugged, but also the catalytically active component falls down, whereby the catalytic performance is not sufficiently exhibited.

Furthermore, Patent Document 4 discloses a technique in which by filling catalysts having a different ratio of bismuth and iron in each reaction tube having two or more reaction zones disposed therein in the axial direction of the tube in such a manner a total amount of bismuth and iron decreases from the raw material gas inlet side toward the outlet side, the sublimation of the molybdenum component is suppressed, thereby producing acrolein and acrylic acid stably over a long period of time.

Furthermore, Patent Document 5 describes a method in which when preparing two or more kinds of complex metal oxide catalysts having a different formulations from each other and stacking two or more layers in the axial direction of the tube, thereby achieving multilayer filling, the catalysts are filled in such a manner that not only the component amount of bismuth relative to molybdenum decreases from the gas inlet side toward the gas outlet side, but also the component amount of iron relative to molybdenum increases from the gas inlet side toward the gas outlet side, even in a reaction under high-load circumstances, the reaction bath temperature is controlled at a low level.

In the foregoing conventional techniques, there is not found any investigation in which in a process of producing, from an alkene, a corresponding unsaturated aldehyde and/or unsaturated carboxylic acid, not only a total yield of the unsaturated aldehyde and/or unsaturated carboxylic acid (the total yield will be hereinafter referred to as "effective yield") itself is improved, but also even in a reaction under high-load circumstances, while controlling the reaction bath temperature at a low level, the production is achieved stably while maintaining the improved effective yield.

Background Art Document

Patent Document

Patent Document 1: JP-A-2001-226302

Patent Document 2: JP-A-H6-192144

Patent Document 3: JP-T-2007-511565

Patent Document 4: JP-A-2001-048817

Patent Document 5: WO-A-2015/008815

Summary of Invention

Problem that Invention is to Solve

An object of the present invention is to provide a technique capable of stably producing, from an alkene, a corresponding unsaturated aldehyde and/or unsaturated carboxylic acid while not only controlling a reaction bath temperature at a low level even under high-load reaction conditions but also improving and maintaining an effective yield.

Means for Solving Problem

In order to solve the above-described problem, in a method of subjecting an alkene to gas-phase catalytic partial oxidation with molecular oxygen by using a multitubular oxidation reactor having a complex metal oxide catalyst filled therein, thereby producing a corresponding unsaturated aldehyde and/or unsaturated carboxylic acid, the present inventors paid attention to the amount of bismuth that is important among constituent elements of the catalyst and made extensive and intensive investigations. As a result, they have found a technique in which when not only two or more catalyst layers each containing a complex metal oxide catalyst are stuck in the axial direction of the tube under specified conditions, thereby achieving multilayer filling, but also the catalyst layer on the most gas outlet side in the tube axis contains a catalyst containing a compound represented by a specified formulation formula, a catalytic performance of the catalyst with high activity and high yield is effectively exhibited, and the catalyst on the raw material gas inlet side becomes high in selectivity and long in life, whereas the catalyst on the raw material gas outlet side becomes conspicuously high in activity, whereby even in a high-load reaction, the reaction bath temperature can be controlled at a low level, the effective yield is improved, and a target product is stably obtained over a long period of time, thereby leading to accomplishment of the present invention.

That is, the present invention relates to:

(1) A method for producing an unsaturated aldehyde and/or an unsaturated carboxylic acid, the method comprising subjecting an alkene to gas-phase catalytic partial oxidation with molecular oxygen by using a multitubular reactor having a complex metal oxide catalyst filled therein, thereby obtaining a corresponding unsaturated aldehyde and/or unsaturated carboxylic acid, wherein

two or more catalyst layers each containing a complex metal oxide catalyst are stuck in an axial direction of the tube, thereby achieving multilayer filling;
a formulation of the complex metal oxide catalyst contained in one catalyst layer is different from a formulation of the complex metal oxide catalyst contained in at least one of other catalyst layers;
a ratio of a component amount of bismuth to a component amount of molybdenum of the catalyst layer on the most gas inlet side in the tube axis is larger than a ratio of a component amount of bismuth to a component amount of molybdenum of the catalyst layer on the most gas outlet side in the tube axis;
in all of the two catalyst layers adjacent to each other, a ratio of a component amount of bismuth to a component amount of molybdenum of the catalyst layer on the gas inlet side in the tube axis is equal to or larger than a ratio of a component amount of bismuth to a component amount of molybdenum of the catalyst layer on the gas outlet side in the tube axis; and

the catalyst layer on the most gas outlet side in the tube axis contains a catalyst containing a compound represented by the following formula (1):

$$Mo_{12}Bi_aFe_bCo_cNi_dX_eY_fZ_gO_h \qquad (1)$$

wherein

X is at least one element selected from the group consisting of magnesium (Mg), calcium (Ca), manganese (Mn), copper (Cu), zinc (Zn), cerium (Ce) and samarium (Sm); Y is at least one element selected from the group consisting of boron (B), phosphorus (P), arsenic (As), antimony (Sb), tungsten (W), silicon (Si) and aluminum (Al); Z is at least one element selected from the group consisting of sodium (Na), potassium (K), rubidium (Rb) and cesium (Cs); a to h represent atomic ratios of the respective components; a = 0.40 or more and less than 0.80; b = 1.0 to 2.5; c = 3.0 to 7.5; d = 2.0 to 3.5; e = 0 to 10; f = 0 to 10; g = 0.01 to 1.0; h is expressed by a numerical value satisfying oxidation states of other elements; d/a is more than 2.5 and 8.8 or less; d/g is 2.0 or more and 350 or less; and a/g is 0.4 or more and less than 80;

(2) The method for producing a compound represented by formula (1) according to (1),

wherein in a step of preparing the compound represented by the formula (1), a molybdenum component raw material is constituted of only an ammonium molybdate, and a weight of water for dissolution is 8.5 times or less relative to a weight of molybdenum contained in the ammonium molybdate; and

a bismuth component raw material is constituted of only bismuth nitrate, a weight of a nitric acid aqueous solution for dissolution is 2.3 times or more relative to a weight of bismuth contained in the bismuth nitrate, and a concentration of nitric acid of the nitric acid aqueous solution for dissolving bismuth nitrate therein is 10% by weight or more;

(3) The method for producing an unsaturated aldehyde and/or an unsaturated carboxylic acid as described in (1), wherein the complex metal oxide catalyst is a spherical coated catalyst in which catalytic active components are supported on a surface of an inert carrier;

(4) The method for producing an unsaturated aldehyde and/or an unsaturated carboxylic acid as described in any one of (1) or (2),

wherein a load of the alkene in the raw material gas to be supplied into the multitubular reactor is 100 times or more (converted in a standard state) relative to a unit catalyst volume per hour;

(5) The method for producing an unsaturated aldehyde and/or an unsaturated carboxylic acid as described in any one of (1) or (3) to (4),

wherein a load of the alkene in the raw material gas to be supplied into the multitubular reactor is 150 times or more (converted in a standard state) relative to a unit catalyst volume per hour;

(6) The method for producing an unsaturated aldehyde and/or an unsaturated carboxylic acid as described in any one of (1) or (3) to (5),

wherein a concentration of the alkene contained in the raw material gas to be supplied into the multitubular reactor is 8.5% by volume or less;

(7) The method for producing an unsaturated aldehyde and/or an unsaturated carboxylic acid according to any one of (1) or (3) to (6),

wherein all of the catalyst layers filled in the multitubular reactor are not diluted by physical mixing of the complex metal oxide catalyst and an inert substance; and

(8) A method for producing acrolein and/or acrylic acid, or methacrolein and/or methacrylic acid, by the method for producing an unsaturated aldehyde and/or an unsaturated carboxylic acid as described in any one of (1) or (3) to (7).

Effects of Invention

In accordance with the present invention, it is possible to stably produce, from an alkene, a corresponding unsaturated aldehyde and/or unsaturated carboxylic acid in an improved effective yield over a long period of time, while controlling the reaction bath temperature at a low level even in a high-load reaction.

Mode for Carrying Out Invention

The present invention is concerned with a method of subjecting an alkene to gas-phase catalytic partial oxidation with molecular oxygen by using a multitubular reactor having a complex metal oxide catalyst filled therein, thereby producing a corresponding unsaturated aldehyde and/or unsaturated carboxylic acid, wherein two or more catalyst layers each containing a complex metal oxide catalyst are stuck in the axial direction of the tube, thereby achieving multilayer filling; a formulation of the complex metal oxide catalyst contained in one catalyst layer is different from a formulation of the complex metal oxide catalyst contained in at least one of other catalyst layers; a ratio of the component amount of bismuth to the component amount of molybdenum of the catalyst layer on the most gas inlet side in the tube axis is larger than a ratio of the component amount of bismuth to the component amount of molybdenum of the catalyst layer on the most gas outlet side in the tube axis; in all of the two catalyst layers adjacent to each other, a ratio of the component amount of bismuth to the component amount of molybdenum of the catalyst layer on the gas inlet side in the tube axis is equal to or larger than a ratio of the component amount of bismuth to the component amount of molybdenum of the catalyst layer on the gas outlet side in the tube axis; and the catalyst layer on the most gas outlet side in the tube axis contains a catalyst containing a compound represented by the formula (1).

Here, as for the constituent element ratios of the compound represented by the formula (1), ratios of bismuth that is one of the catalyst main components, and nickel and an alkali metal, each of which largely influences the activity, are important. When d/a that is a ratio of nickel to bismuth is more than 2.5 and 8.8 or less, d/g that is a ratio of nickel to the alkali metal is 2.0 or more and 350 or less, and a/g that is a ratio of bismuth to the alkali metal is 0.4 or more and less than 80, an excellent catalyst which is high in selectivity and yield of a target product is provided. In addition, when the amount of bismuth is 0.40 or more and less than 0.80, and preferably 0.40 or more and less than 0.75, a higher yield is obtained.

The catalyst of the present invention is filled in the catalyst layer on the most gas outlet side in the tube axis, and for example, in the case of two-layer filling, the catalyst containing the compound represented by the formula (1) may be filled in the catalyst layer on the gas outlet side in the tube axis, whereas an arbitrary catalyst may be filled in the catalyst layer on the gas inlet side. The arbitrary catalyst which is contained in the catalyst layer on the gas inlet side is a catalyst containing molybdenum and bismuth, and for example, a catalyst containing a compound represented by the following formula (2) can be used.

$$Mo_{12}Bi_aFe_bCo_cNi_dX_eY_fZ_gO_h \qquad (2)$$

In the formula, X is at least one element selected from the group consisting of magnesium (Mg), calcium (Ca), manganese (Mn), copper (Cu), zinc (Zn), cerium (Ce) and samarium (Sm); Y is at least one element selected from the group consisting of boron (B), phosphorus (P), arsenic (As), antimony (Sb), tungsten (W), silicon (Si) and aluminum (Al); Z is at least one clement selected from the group consisting of sodium (Na), potassium (K), rubidium (Rb) and cesium (Cs); a to h represent atomic ratios of the respective components; a = 0.80 or more and 2.0 or less; b = 1.0 to 2.5; c = 3.0 to 7.5; d = 2.0 to 3.5; e = 0 to 10; f= 0 to 10; g = 0.01 to 1.0; h is expressed by the numerical value satisfying the oxidation states of other elements; d/a is more than 2.5 and 8.8 or less; d/g is 2.0 or more and 350 or less; and a/g is 0.4 or more and less than 80.

In this case, the catalysts are filled in the tube axis in such a manner that a ratio of the component amount of bismuth to the component amount of molybdenum of the catalyst layer on the gas inlet side is larger than a ratio of the component amount of bismuth to the component amount of molybdenum of the catalyst layer on the gas outlet side.

In addition, for example, in the case of three-layer filling, the catalyst containing the compound represented by the formula (1) may be filled in the catalyst layer on the most gas outlet side in the tube axis, whereas an arbitrary catalyst may be filled in the catalyst layer on the most gas inlet side and the second catalyst layer from the gas inlet side in the tube axis. The arbitrary catalyst which is contained in the catalyst layer on the most gas inlet side and the second catalyst layer from the gas inlet side in the tube axis is a catalyst containing molybdenum and bismuth, and for example, a catalyst containing the compound represented by the formula (2) can be used. In this case, the catalysts are filled in the tube axis in such a manner that a ratio of the component amount of bismuth to the component amount of molybdenum of the catalyst layer on the most gas inlet side in the tube axis is larger than a ratio of the component amount of bismuth to the component amount of molybdenum of the catalyst layer on the most gas outlet side in the tube axis, and even in all of the two catalyst layers adjacent to each other, a ratio of the component amount of bismuth to the component amount of molybdenum of the catalyst layer on the gas inlet side in the tube axis is equal to or larger than a ratio of the component amount of bismuth to the component amount of molybdenum of the catalyst layer on the gas outlet side in the tube axis.

In addition, in any one or all of layers in multilayer filling, the adaptation can also be achieved by mixing with an inert substance and/or adjusting the particle diameter, if desired.

The catalyst which is used in the present invention (hereinafter referred to as "catalyst of the present invention") can be prepared through the following steps.

Step a): Preparation

In general, starting raw materials of respective elements constituting the catalyst are not particularly limited. As the molybdenum component raw material, molybdenum oxides, such as molybdenum trioxide, molybdic acid or salts thereof, such as molybdic acid and an ammonium molybdate, molybdenum-containing heteropolyacids or salts thereof, such as phosphomolybdic acid and silicomolybdic acid, and the like can be used; however, more preferably, in the case of using an ammonium molybdate, a high-performance catalyst tends to be obtained. In particular, though the ammonium molybdate includes plural kinds of compounds, such as ammonium dimolybdate, ammonium tetramolybdate, ammonium heptamolybdate, etc., among those, the case of using ammonium heptamolybdate is the most preferred. As the bismuth component raw material, bismuth salts, such as bismuth nitrate, bismuth subcarbonate, bismuth sulfate, bismuth acetate, etc., bismuth trioxide, metallic bismuth, and the like can be used; however, more preferably, in the case of using bismuth nitrate, a high-performance catalyst tends to be obtained. As for raw materials of iron, cobalt, nickel, and other elements, oxides, or nitrates, carbonates, organic acid salts, hydroxides, and the like, each of which may become an oxide by heat, or mixtures thereof, can be generally used. For example, the iron component raw material and the cobalt component raw material and/or the nickel component raw material are dissolved in a desired ratio in water and mixed under a condition at 10 to 80°C; the mixture is mixed with an aqueous solution or slurry of the separately prepared molybdenum component raw material and Z component raw material under a condition at 20 to 90°C; after heating and stirring the resulting mixture for about 1 hour under a condition at 20 to 90°C, an aqueous solution having the bismuth component raw material dissolved therein and optionally the X component raw material and the Y component raw material are added, thereby obtaining an aqueous solution or slurry containing the catalyst components. In the case of adding the X component and the Y component, it is preferred to use, as the raw materials, oxides, or nitrates, carbonates, organic acid salts, hydroxides, and the like, each of which may become an oxide upon ignition, or mixtures thereof, and as for the addition amounts, it is preferred to add the X component and the Y component in the preparation step such that when the content of molybdenum is defined as 12, the contents of the X component and the Y component fall within a range of 0.05 to 10 in terms of atomic ratios. Such aqueous solutions or slurries are hereinafter collectively called "liquid preparation (A)". Here, the liquid preparation (A) is not always required to contain all of the catalyst constituent elements, and a part of those elements or a part of the amounts thereof may be added in the sequent step or steps. In addition, on the occasion of preparing the liquid preparation (A), when the amount of water for dissolving each of the component raw materials, or in the case of adding an acid, such as sulfuric acid, nitric acid, hydrochloric acid, tartaric acid, acetic acid, etc., for the purpose of dissolution, the acid concentration in the aqueous solution sufficient for dissolution of the raw materials is not suitable for the preparation within a range of, for example, 5% by weight to 99% by weight, there would be a case where the form of the liquid preparation (A) becomes a clay-like lump. In this case, an excellent catalyst is not obtained. The form of the liquid preparation (A) is preferably an aqueous solution or slurry from the standpoint that an excellent catalyst is obtained.

In the step of preparing the compound represented by the foregoing formula (1), it is preferred that the molybdenum component raw material is constituted of only an ammonium molybdate, and the weight of water for dissolution is 8.5 times or less relative to the weight of molybdenum contained in the ammonium molybdate; the bismuth component raw material is constituted of only bismuth nitrate, the weight of a nitric acid aqueous solution for dissolution is 2.3 times or more relative to the weight of bismuth contained in the bismuth nitrate, and the concentration of nitric acid of the nitric acid aqueous solution for dissolving bismuth nitrate is 10% by weight or more; and the resulting catalyst is filled in the catalyst layer on the most gas outlet side in the tube axis. In order to take into account the reaction efficiency, the balance of temperature distribution, and so on, the catalyst of the present invention may be subjected to multilayer filling, and in multilayer filling, the catalyst of the present invention may also be filled in combination with a catalyst having other formulation.

Step b): Drying

Subsequently, the liquid preparation (A) obtained above is dried to form a dry powder. The drying method is not particularly limited so long as it is a method capable of completely drying the liquid preparation (A); however, examples thereof include drum drying, freeze drying, spray drying, evaporation to dryness, and the like. Of these, spray drying in which the slurry can be dried into a powder or granule within a short period of time is especially preferred in the present invention. Although the drying temperature of spray drying varies depending upon the concentration of slurry, the liquid sending speed, or the like, it is approximately 70 to 150°C in terms of a temperature at the outlet of a drying machine. In addition, it is preferred to perform drying such that an average particle diameter of the dry powder obtained on that occasion is 10 to 700 $\mu$m. There is thus obtained a dry powder (B).

Step c): Preliminary Calcination

When the obtained dry powder (B) is calcined under air circulation at 200 to 600°C, and preferably 300 to 600°C, molding properties, mechanical strength, and catalytic performance of the resulting catalyst tend to be improved. A calcination time is preferably 1 to 12 hours. There is thus obtained a preliminarily calcined powder (C).

Step d): Molding

Although the molding method is not particularly limited, on the occasion of molding in a cylindrical or annular form, a method using a tablet molding machine, an extrusion molding machine, or the like is preferred. The case of molding in a spherical form is more preferred, and the preliminarily calcined powder (C) may be molded in a spherical form by using a molding machine; however, a method of supporting the preliminarily calcined powder (C) (including a molding auxiliary agent and a strength improver, if desired) on a carrier, such as an inert ceramic, etc., is preferred. Here, as for the supporting method, a tumbling granulation method, a method using a centrifugal flow coating apparatus, a wash coating method, and the like arc widely known, and the supporting method is not particularly limited so long as it is a method capable of uniformly supporting the preliminarily calcined powder (C) on the carrier. However, in the case of taking into account the production efficiency of the catalyst or the performance of the prepared catalyst, more preferably, a method in which using an apparatus having a flat or uneven disk in a bottom of a fixed cylindrical vessel, a carrier charged within the vessel is vigorously agitated by means of rotation motion and revolution motion of the carrier itself by rotating the disk at a high speed, and the preliminarily calcined powder (C) and optionally a molding auxiliary agent and/or a strength improver, are added thereto, thereby supporting the powder components on the carrier is preferred. It is to be noted that on the occasion of supporting, it is preferred to use a binder. Specific examples of the binder which may be used include water, ethanol, methanol, propanol, a polyhydric alcohol, polyvinyl alcohol that is a polymer-based binder, a silica sol aqueous solution that is an inorganic binder, and the like; ethanol, methanol, propanol, and a polyhydric alcohol are preferred; and a diol, such as ethylene glycol, etc., a triol, such as glycerin, etc., and the like are more preferred. By using an appropriate amount of a glycerin aqueous solution, the molding properties become good, and a high-performance catalyst having high mechanical strength is obtained. Specifically, in the case of using an aqueous solution having a glycerin concentration of 5% by weight or more, a catalyst having an especially high performance tends to be obtained. The use amount of such a binder is typically 2 to 80 parts by weight based on 100 parts by weight of the preliminarily calcined powder (C). As for the inert carrier, a carrier having a diameter of about 2 to 8 mm is generally used, and the preliminarily calcined powder (C) is supported thereon. Its supporting rate is determined taking into account a use condition of the catalyst, for example, a reaction condition, such as a space velocity of the reaction raw materials, raw material concentrations, etc., and it is typically 20 to 80% by weight. Here, the supporting rate is expressed according to the following formula (3).

$$\text{Supporting rate (\% by weight)} = 100 \times [(\text{Weight of preliminarily calcined powder (C)} \text{ used for molding})/\{(\text{Weight of preliminarily calcined powder (C) used for molding}) + (\text{Weight of inert carrier used for molding})\}] \qquad (3)$$

There is thus obtained a molded body (D).

Step e): Calcination

By calcining the molded body (D) at a temperature of 200 to 600°C for about 1 to 12 hours, the catalytic activity and effective yield tend to be improved. The calcination temperature is preferably 400°C or higher and 600°C or lower, and more preferably 500°C or higher and 600°C or lower. Since the optimum calcination temperature of the catalysts which are different in an atomic ratio from each other is different, in the case of changing the atomic ratio of the catalyst, when the molded body (D) is calcined at a temperature that is optimum in that atomic ratio, a high-performance catalyst tends to be obtained. Air is simple and easy and preferred as the gas to be circulated; however, besides, it is also possible to use nitrogen or carbon dioxide as an inert gas, or a nitrogen oxide-containing gas, an ammonia-containing gas, a hydrogen gas, or a mixture thereof for the purpose of rendering the system into a reducing atmosphere. There is thus obtained a catalyst (E). The mechanical strength of the catalyst (E) is also largely influenced by the atomic ratios of the catalyst formulation. That is, the mechanical strength of the catalyst (E) is influenced by the kind of a compound to be formed by regulating the atomic ratios, or the matter that even in the same compound, the phase form of a crystal structure is different. In addition, the diameter of the complex metal oxide particle formed in the preparation step or drying step or the geometric structure of the particle, and the coagulation form thereof change, and therefore, the mechanical strength of the catalyst (E) is also influenced by changes in micro physical properties, such as strength of the compound crystal in the complex metal oxide, or macro physical properties, for example, the particle size distribution of the preliminarily calcined powder. Overall physical properties including not only the preparation method of each step but also the influence of the atomic ratios determine the mechanical strength of the ultimately prepared catalyst.

The catalyst containing the compound represented by the foregoing formula (1) as obtained in this method is filled in the catalyst layer on the most gas outlet side in the tube axis and can be used in a step of subjecting propylene as the raw material gas to gas-phase catalytic partial oxidation with molecular oxygen or a molecular oxygen-containing gas to produce acrolein and acrylic acid, or a step of subjecting isobutylene as the raw material gas and tertiary butanol to gas-phase catalytic partial oxidation with molecular oxygen or a molecular oxygen-containing gas to produce methacrolein and methacrylic acid. For example, the gas-phase catalytic partial oxidation is carried out by introducing a mixed gas composed of 1 to 10% by volume of an alkene, 5 to 18% by volume of molecular oxygen, 0 to 60% by volume of steam, and 20 to 70% by volume of an inert gas, for example, nitrogen, carbon dioxide, etc., in terms of a raw material gas formulation onto the catalyst prepared above at a temperature ranging from 250 to 450°C under a pressure of atmospheric pressure to 10 atms under a supply load of the alkene of 60 to 200 $hr^{-1}$ in terms of a space velocity. As the alkene supply load becomes high, an improving effect of the effective yield described in the present invention becomes remarkable. The gas-phase catalytic partial oxidation is preferably carried out in the amount of the alkene in the mixed gas of 8.5% by volume or less under a supply load of the alkene of 100 $hr^{-1}$ or more, and more preferably 150 $hr^{-1}$ or more in terms of a space velocity. Here, an alkene space velocity ($SV_0$) means the raw material load, and for example, the introduction at an alkene space velocity ($SV_0$) of 100 $hr^{-1}$ means that the gas-phase catalytic partial oxidation reaction is carried out while supplying the alkene in an amount of 100 times (converted in a standard state) relative to a unit catalyst volume per hour. The alkene as referred to in the present invention also includes an alcohol capable of producing an alkene in its intramolecular dehydration reaction, for example, tertiary butanol.

Examples

Examples are hereunder described by reference to specific examples, but it should be construed that the present invention is not limited to these Examples so long as the gist of the present invention is not deviated.

Catalyst 1

2,000 parts by weight of ammonium heptamolybdate was completely dissolved in 7,600 parts by weight of pure water warmed at 60°C. Subsequently, 9.2 parts by weight of potassium nitrate was dissolved in 104.1 parts by weight of pure water and added to the above-described solution. Subsequently, 686.4 parts by weight of ferric nitrate, 1,428.8 parts by weight of cobalt nitrate, and 768.6 parts by weight of nickel nitrate were dissolved in 1,528.4 mL of pure water warmed at 60°C. These solutions were gradually mixed while stirring. Subsequently, a solution prepared by adding 198.2 parts by weight of nitric acid (60% by weight) to 825.2 mL of pure water, to which was then added 778.4 parts by weight of bismuth nitrate and completely dissolved, was added to the above-described solution, followed by stirring and mixing. This slurry was dried by the spray drying method, and the resulting dry powder was preliminarily calcined at a maximum temperature 440°C for 6 hours. Crystalline cellulose was added in a proportion of 5% by weight relative to the preliminarily calcined powder and thoroughly mixed. Subsequently, the mixture was supported and molded in a spherical form at a supporting rate of 50% by weight on an inert spherical carrier composed mainly of silica and alumina and having a diameter of 4.5 mm by using a 30% by weight glycerin solution as a binder by the tumbling granulation method. Subsequently, calcination was carried out at 560°C for 4 hours, thereby obtaining Spherical Catalyst 1 having an average particle diameter of 5.2 mm. The catalyst calculated from the charged raw materials was found to be a complex metal oxide having the following atomic ratios.

d/a= 1.6, d/g = 28, a/g = 17
Mo:Bi:Fe:Co:Ni:K = 12:1.7:1.8:5.2:2.8:0.10

Catalyst 2

2,000 parts by weight of ammonium heptamolybdate was completely dissolved in 7,600 parts by weight of pure water (in a weight of 7.0 times the weight of molybdenum) warmed at 60°C. Subsequently, 4.4 parts by weight of potassium nitrate was dissolved in 50.1 parts by weight of pure water and added to the above-described solution. Subsequently, 762.7 parts by weight of ferric nitrate, 1,786.0 parts by weight of cobalt nitrate, and 823.5 parts by weight of nickel nitrate were dissolved in 1,787.3 mL of pure water warmed at 60°C. These solutions were gradually mixed while stirring. Subsequently, a solution prepared by adding 320.5 parts by weight of bismuth nitrate to a nitric acid aqueous solution (in a weight of 2.3 times the weight of bismuth in bismuth nitrate to be dissolved) which had been prepared by adding 81.6 parts by weight of nitric acid (60% by weight) to 339.8 mL of pure water, thereby regulating a nitric acid concentration to 12% by weight and then completely dissolving was added to the above-described solution and mixed with stirring. This slurry was dried by the spray drying method, and the resulting dry powder was preliminarily calcined so as to keep the maximum temperature at 440°C for 4 hours. Crystalline cellulose was added in proportion of 5% by weight relative to the preliminarily calcined powder and thoroughly mixed. Thereafter, the mixture was supported and molded in a spherical form at a supporting rate of 50% by weight on an inert spherical carrier by using a 30% by weight glycerin solution as a binder by the tumbling granulation method. Subsequently, calcination was carried out so as to be held at a maximum temperature of 520°C for 4 hours, thereby obtaining Spherical Catalyst 2 having an average particle diameter of 5.2 mm. The catalyst calculated from the charged raw materials was found to be a complex metal oxide having the following atomic ratios.

d/a = 4.3, d/g = 60, a/g = 14

Mo:Bi:Fe:Co:Ni:K = 12:0.7:2.0:6.5:3.0:0.05

Catalyst 3

2,000 parts by weight of ammonium heptamolybdate was completely dissolved in 7,600 parts by weight of pure water warmed at 60°C. Subsequently, 5.5 parts by weight of cesium nitrate was dissolved in 62.2 parts by weight of pure water and added to the above-described solution. Subsequently, 762.7 parts by weight of ferric nitrate, 1,786.0 parts by weight of cobalt nitrate, and 823.5 parts by weight of nickel nitrate were dissolved in 1,787.3 mL of pure water warmed at 60°C. These solutions were gradually mixed while stirring. Subsequently, a solution prepared by adding 116.6 parts by weight of nitric acid (60% by weight) to 485.5 mL of pure water, to which was then added 457.9 parts by weight of bismuth nitrate and completely dissolved, was added to the above-described solution, followed by stirring and mixing. This slurry was dried by the spray drying method, and the resulting dry powder was preliminarily calcined at a maximum temperature 440°C for 6 hours. Crystalline cellulose was added in a proportion of 5% by weight relative to the preliminarily calcined powder and thoroughly mixed. Thereafter, the mixture was supported and molded in a spherical form at a supporting rate of 50% by weight on an inert spherical carrier composed mainly of silica and alumina and having a diameter of 4.5 mm by using a 30% by weight glycerin solution as a binder by the tumbling granulation method. Subsequently, calcination was carried out at 540°C for 4 hours, thereby obtaining Spherical Catalyst 3 having an average particle diameter of 5.2 mm. The catalyst calculated from the charged raw materials was found to be a complex metal oxide having the following atomic ratios.

d/a = 3.0, d/g = 100, a/g = 33

Mo:Bi:Fe:Co:Ni:Cs = 12:1.0:2.0:6.5:3.0:0.03

Catalyst 4

Catalyst 4 was obtained in the same manner as in Catalyst 2, except that only the calcination step temperature after molding was changed to 540°C.

Catalyst 5

2,000 parts by weight of ammonium heptamolybdate was completely dissolved in 7,600 parts by weight of pure water warmed at 60°C. Subsequently, 4.4 parts by weight of potassium nitrate was dissolved in 50.1 parts by weight of pure water and added to the above-described solution. Subsequently, 762.7 parts by weight of ferric nitrate, 1,786.0 parts by weight of cobalt nitrate, and 823.5 parts by weight of nickel nitrate were dissolved in 1,787.3 mL of pure water warmed at 60°C. These solutions were gradually mixed while stirring. Subsequently, a solution prepared by adding 116.6 parts by weight of nitric acid (60% by weight) to 485.5 mL of pure water, to which was then added 457.9 parts by weight of bismuth nitrate and completely dissolved, was added to the above-described solution, followed by stirring and mixing. This slurry was dried by the spray drying method, and the resulting dry powder was preliminarily calcined at a maximum temperature 440°C for 6 hours. Crystalline cellulose was added in a proportion of 5% by weight relative to the preliminarily calcined powder and thoroughly mixed. Thereafter, the mixture was supported and molded in a spherical form at a supporting rate of 50% by weight on an inert spherical carrier composed mainly of silica and alumina and having a diameter of 4.5 mm by using a 30% by weight glycerin solution as a binder by the tumbling granulation method. Subsequently, calcination was carried out at 550°C for 4 hours, thereby obtaining Spherical Catalyst 5 having an average particle diameter of 5.2 mm. The catalyst calculated from the charged raw materials was found to be a complex metal oxide having the following atomic ratios.

d/a = 3.0, d/g = 60, a/g = 20

Mo:Bi:Fe:Co:Ni:K = 12:1.0:2.0:6.5:3.0:0.05

Catalyst 6

2,000 parts by weight of ammonium heptamolybdate was completely dissolved in 7,600 parts by weight of pure water warmed at 60°C. Subsequently, 9.2 parts by weight of potassium nitrate was dissolved in 104.1 parts by weight of pure water and added to the above-described solution. Subsequently, 877.1 parts by weight of ferric nitrate, 1,373.9 parts by weight of cobalt nitrate, and 768.6 parts by weight of nickel nitrate were dissolved in 1,600.4 mL of pure water warmed at 60°C. These solutions were gradually mixed while stirring. Subsequently, a solution prepared by adding 151.6 parts by weight of nitric acid (60% by weight) to 631.1 mL of pure water, to which was then added 595.3 parts by weight of bismuth nitrate and completely dissolved, was added to the above-described solution, followed by stirring and mixing. This slurry was dried by the spray drying method, and the resulting dry powder was preliminarily calcined at a maximum temperature 440°C for 6 hours. Crystalline cellulose was added in a proportion of 5% by weight relative to the preliminarily calcined powder and thoroughly mixed. Thereafter, the mixture was supported and molded in a spherical form at a supporting rate of 50% by weight on an inert spherical carrier composed mainly of silica and alumina and having a diameter of 4.5 mm by using a 30% by weight glycerin solution as a binder by the tumbling granulation method. Subsequently, calcination was carried out at 530°C for 4 hours, thereby obtaining Spherical Catalyst 6 having an average particle diameter of 5.2 mm. The catalyst calculated from the charged raw materials was found to be a complex metal oxide having the following atomic ratios.

d/a = 2.2, d/g = 28, a/g = 13

Mo:Bi:Fe:Co:Ni:K = 12:1.3:2.3:5.0:2.8:0.10

Example 1

A gas-phase catalytic oxidation reaction of propylene was carried out by using Catalyst 1 and Catalyst 2, thereby determining catalytic performances represented by a propylene conversion, an acrolein yield (A), an acrylic acid yield (B), and an effective yield (A + B). Catalyst 1 was filled in a filling length of 1,500 mm on the raw material gas inlet side of a stainless steel-made reaction tube having a diameter of 25.2 mm, in which a thermocouple protection tube having an outer diameter of 3.2 mm was placed; and the above-described Catalyst 2 was filled in a filling length of 2,000 mm on the raw material gas outlet side. A mixed gas composed of 7.4% by volume of propylene, 63.2% by volume of air, 7.4% by volume of steam, and 22.1% by volume of nitrogen as a raw material gas was introduced at a propylene space velocity ($SV_0$) of 165 $hr^{-1}$ from the reaction tube inlet, a pressure on the gas outlet side was adjusted at 95 kPaG, and the gas-phase catalytic partial oxidation reaction of propylene was carried out. The results when the propylene conversion at the moment when the reaction elapsed about 300 hours after commencement of the reaction became 98% are shown in Table 1.

Example 2

A gas-phase catalytic oxidation reaction of propylene was carried out by using Catalyst 3 and Catalyst 4, thereby determining catalytic performances represented by a propylene conversion, an acrolein yield (A), an acrylic acid yield (B), and an effective yield (A + B). Catalyst 3 was filled in a filling length of 1,200 mm on the raw material gas inlet side of a stainless steel-made reaction tube having a diameter of 27.2 mm, in which a thermocouple protection tube having an outer diameter of 6.0 mm was placed; and the above-described Catalyst 4 was filled in a filling length of 1,700 mm on the raw material gas outlet side. A mixed gas composed of 8.2% by volume of propylene, 64.0% by volume of air, 24.4% by volume of steam, and 3.4% by volume of nitrogen as a raw material gas was introduced at a propylene space velocity ($SV_0$) of 140 $hr^{-1}$ from the reaction tube inlet, a pressure on the gas outlet side was adjusted at 80 kPaG, and the gas-phase catalytic partial oxidation reaction of propylene was carried out. The results when the propylene conversion at the moment when the reaction elapsed about 300 hours after commencement of the reaction became 98% are shown in Table 1.

Example 3

The oxidation reaction of propylene was carried out in the same manner as in Example 2, except that the filling was changed to diluted three-layer filling with Catalyst 3 and Catalyst 4. The diluted three-layer filling was carried out in such a manner that the catalyst concentration became high in the order of the upper layer to the intermediate layer to the lower layer, from the gas inlet side toward the gas outlet side. As for the upper layer, Catalyst 3 was diluted with an inert substance composed mainly of silica and alumina and having an average particle diameter of 5.2 mm such that the catalyst weight was 90% by weight, and then filled in a filling length of 650 mm. Subsequently, as for the intermediate layer, Catalyst 3 was filled such that the catalyst weight was 100% by weight; and that the filling length was 650 mm. Finally, as for the lower layer, Catalyst 4 was filled such that the catalyst weight was 100% by weight; and that the filling length was 1,600 mm. The results when the propylene conversion at the moment when the reaction elapsed about 300 hours after commencement of the reaction became 98% are shown in Table 1.

Comparative Example 1

The oxidation reaction of propylene was carried out in the same manner as in Example 1, except for changing Catalyst 2 to Catalyst 5. The results when the propylene conversion at the moment when the reaction elapsed about 300 hours after commencement of the reaction became 98% are shown in Table 1.

Comparative Example 2

The oxidation reaction of propylene was carried out in the same manner as in Example 2, except for changing Catalyst 4 to Catalyst 5. The results when the propylene conversion at the moment when the reaction elapsed about 300 hours after commencement of the reaction became 98% are shown in Table 1.

Comparative Example 3

The oxidation reaction of propylene was carried out in the same manner as in Example 2, except that the filling was changed to diluted three-layer filling with Catalyst 6. The diluted three-layer filling was carried out in such a manner that the catalyst concentration became high in the order of the upper layer to the intermediate layer to the lower layer, from the gas inlet side toward the gas outlet side. As for the upper layer, the catalyst was diluted with an inert substance composed mainly of silica and alumina and having an average particle diameter of 5.2 mm such that the catalyst weight was 70% by weight, and then filled in a filling length of 700 mm. Subsequently, as for the intermediate layer, the catalyst was diluted with the above-described inert substance such that the catalyst weight was 80% by weight, and then filled in a filling length of 700 mm. Finally, as for the lower layer, the catalyst was filled such that the catalyst weight was 100% by weight; and that the filling length was 1,500 mm. The results when the effective yield at the moment when the reaction elapsed about 300 hours after commencement of the reaction became maximum are shown in Table 1. It is to be noted that in Comparative Example 3, in the case where the reaction was carried out such that the propylene conversion became 98%, the hot spot temperature became excessively high, so that it was difficult to keep the stable reaction state.

Table 1

|  | Catalyst | Reaction bath temperature (°C) | Maximum reaction temperature (°C) | Effective yield (A+B) (%) |
|---|---|---|---|---|
| Example 1 | Catalyst 1, Catalyst 2 | 326 | 421 | 90.7 |
| Example 2 | Catalyst 3, Catalyst 4 | 328 | 425 | 90.3 |
| Example 3 | Catalyst 3, Catalyst 4 | 327 | 393 | 90.4 |
| Comparative Example 1 | Catalyst 1, Catalyst 5 | 331 | 420 | 90.2 |
| Comparative Example 2 | Catalyst 3, Catalyst 5 | 326 | 406 | 90.0 |
| Comparative Example 3 | Catalyst 6 | 330 | 439 | 89.5 |
| Effective yield = (Acrolein yield, A%) + (Acrylic acid yield, B%) | | | | |

In the light of the above, by using the technique of the present invention, even in a high-load reaction, a target product can be obtained stably over a long period of time while not only controlling the reaction bath temperature at a low level but also improving the effective yield.

It is to be noted that the present application is based on a Japanese patent application filed on July 10, 2015 (Japanese Patent Application No. 2015-138375).

Industrial Applicability

The present invention is useful for producing an unsaturated aldehyde and/or an unsaturated carboxylic acid.

Claims

1. A method for producing an unsaturated aldehyde and/or an unsaturated carboxylic acid, the method comprising subjecting an alkene to gas-phase catalytic partial oxidation with molecular oxygen by using a multitubular reactor having a complex metal oxide catalyst filled therein, thereby obtaining a corresponding unsaturated aldehyde and/or unsaturated carboxylic acid, wherein

two or more catalyst layers each containing a complex metal oxide catalyst are stuck in an axial direction of the tube, thereby achieving multilayer filling;

a formulation of the complex metal oxide catalyst contained in one catalyst layer is different from a formulation of the complex metal oxide catalyst contained in at least one of other catalyst layers;

a ratio of a component amount of bismuth to a component amount of molybdenum of the catalyst layer on the most gas inlet side in the tube axis is larger than a ratio of a component amount of bismuth to a component amount of molybdenum of the catalyst layer on the most gas outlet side in the tube axis;

in all of the two catalyst layers adjacent to each other, a ratio of a component amount of bismuth to a component amount of molybdenum of the catalyst layer on the gas inlet side in the tube axis is equal to or larger than a ratio of a component amount of bismuth to a component amount of molybdenum of the catalyst layer on the gas outlet side in the tube axis; and

the catalyst layer on the most gas outlet side in the tube axis contains a catalyst containing a compound represented by the following formula (1):

$$Mo_{12}Bi_aFe_bCo_cNi_dX_eY_fZ_gO_h \qquad (1)$$

wherein

X is at least one element selected from the group consisting of magnesium (Mg), calcium (Ca), manganese (Mn), copper (Cu), zinc (Zn), cerium (Ce) and samarium (Sm); Y is at least one element selected from the group consisting of boron (B), phosphorus (P), arsenic (As), antimony (Sb), tungsten (W), silicon (Si) and aluminum (Al); Z is at least one element selected from the group consisting of sodium (Na), potassium (K), rubidium (Rb) and cesium (Cs); a to h represent atomic ratios of the respective components; $a = 0.40$ or more and less than $0.80$; $b = 1.0$ to $2.5$; $c = 3.0$ to $7.5$; $d = 2.0$ to $3.5$; $e = 0$ to $10$; $f = 0$ to $10$; $g = 0.01$ to $1.0$; h is expressed by a numerical value satisfying oxidation states of other elements; $d/a$ is more than $2.5$ and $8.8$ or less; $d/g$ is $2.0$ or more and $350$ or less; and $a/g$ is $0.4$ or more and less than $80$.

2. A method for producing the compound represented by formula (1) according to claim 1,

wherein in a step of preparing the compound represented by the formula (1), a molybdenum component raw material is constituted of only an ammonium molybdate, and a weight of water for dissolution is 8.5 times or less relative to a weight of molybdenum contained in the ammonium molybdate; and

a bismuth component raw material is constituted of only bismuth nitrate, a weight of a nitric acid aqueous solution for dissolution is 2.3 times or more relative to a weight of bismuth contained in the bismuth nitrate, and a concentration of nitric acid of the nitric acid aqueous solution for dissolving bismuth nitrate therein is 10% by weight or more.

3. The method for producing an unsaturated aldehyde and/or an unsaturated carboxylic acid according to claim 1,

wherein the complex metal oxide catalyst is a spherical coated catalyst in which catalytic active components are supported on a surface of an inert carrier.

4. The method for producing an unsaturated aldehyde and/or an unsaturated carboxylic acid according to any one of claims 1 or 3,

wherein a load of the alkene in the raw material gas to be supplied into the multitubular reactor is 100 times or more (converted in a standard state) relative to a unit catalyst volume per hour.

5. The method for producing an unsaturated aldehyde and/or an unsaturated carboxylic acid according to any one of claims 1 or 3 to 4,

wherein a load of the alkene in the raw material gas to be supplied into the multitubular reactor is 150 times or more (converted in a standard state) relative to a unit catalyst volume per hour.

6. The method for producing an unsaturated aldehyde and/or an unsaturated carboxylic acid according to any one of claims 1 or 3 to 5,

wherein a concentration of the alkene contained in the raw material gas to be supplied into the multitubular reactor is 8.5% by volume or less.

7. The method for producing an unsaturated aldehyde and/or an unsaturated carboxylic acid according to any one of claims 1 or 3 to 6,

wherein all of the catalyst layers filled in the multitubular reactor are not diluted by physical mixing of the complex metal oxide catalyst and an inert substance.

8. A method for producing acrolein and/or acrylic acid, or methacrolein and/or methacrylic acid, by the method for producing an unsaturated aldehyde and/or an unsaturated carboxylic acid according to any one of claims 1 or 3 to 7.


**Patentansprüche**

1. Verfahren zur Herstellung eines ungesättigten Aldehyds und/oder einer ungesättigten Carbonsäure, wobei das Verfahren das Unterwerfen eines Alkens einer katalytischen partiellen Gasphasen-Oxidation mit molekularem Sauerstoff unter Verwendung eines mehrröhrigen Reaktors, der einen darin gefüllten komplexen Metalloxidkatalysator aufweist, und dadurch das Erhalten eines entsprechenden ungesättigten Aldehyds und/oder einer ungesättigten Carbonsäure umfasst, wobei

zwei oder mehr Katalysatorschichten, die jeweils einen komplexen Metalloxidkatalysator enthalten, in axialer Richtung des Rohres verklebt sind, wodurch eine Mehrschichtfüllung erreicht wird;

sich eine Formulierung des in einer Katalysatorschicht enthaltenen komplexen Metalloxidkatalysators von einer Formulierung des in mindestens einer der anderen Katalysatorschichten enthaltenen komplexen Metalloxidkatalysators unterscheidet;

ein Verhältnis einer Komponentenmenge von Wismut zu einer Komponentenmenge von Molybdän der Katalysatorschicht auf der meisten Gaseinlassseite in der Rohrachse größer ist als ein Verhältnis einer Komponentenmenge von Wismut zu einer Komponentenmenge von Molybdän der Katalysatorschicht auf der meisten Gasauslassseite in der Rohrachse;

in allen der zwei zueinander angrenzenden Katalysatorschichten, ein Verhältnis einer Komponentenmenge von Wismut zu einer Komponentenmenge von Molybdän der Katalysatorschicht auf der Gaseinlassseite in der Rohrachse gleich oder größer ist als ein Verhältnis einer Komponentenmenge von Wismut zu einer Komponentenmenge von Molybdän der Katalysatorschicht auf der Gasauslassseite in der Rohrachse; und

die Katalysatorschicht auf der meisten Gasauslassseite in der Rohrachse einen Katalysator enthält, der eine Verbindung enthält, die durch die folgende Formel (1) dargestellt wird:

$$Mo_{12}Bi_aFe_bCo_cNi_dX_eY_fZ_gO_h \qquad (1)$$

wobei

X mindestens ein Element ist, ausgewählt aus der Gruppe bestehend aus Magnesium (Mg), Calcium (Ca), Mangan (Mn), Kupfer (Cu), Zink (Zn), Cer(Ce) und Samarium (Sm); Y mindestens ein Element ist, ausgewählt aus der Gruppe bestehend aus Bor (B), Phosphor (P), Arsen (As), Antimon (Sb), Wolfram (W), Silizium (Si) und Aluminium (Al); Z mindestens ein Element ist, ausgewählt aus der Gruppe bestehend aus Natrium (Na), Kalium (K), Rubidium (Rb) und Cäsium (Cs); a bis h Atomverhältnisse der jeweiligen Komponenten darstellen; a = 0,40 oder mehr und weniger als 0,80; b= 1,0 bis 2,5; c = 3,0 bis 7,5; d = 2,0 bis 3,5; e = 0 bis 10; f = 0 bis 10; g = 0,01 bis 1,0; h durch einen Zahlenwert ausgedrückt wird, der Oxidationszustände anderer Elemente erfüllt; d/a mehr als 2,5 und 8,8 oder weniger ist; d/g 2,0 oder mehr und 350 oder weniger ist; und a/g 0,4 oder mehr und weniger als 80 ist.

2. Verfahren zur Herstellung der durch die Formel (1) dargestellten Verbindung nach Anspruch 1, wobei in einem Schritt der Herstellung der durch die Formel (1) dargestellten Verbindung ein Molybdän-Komponenten-Rohmaterial nur aus einem Ammoniummolybdat besteht, und ein Gewicht von Wasser zum Lösen das 8,5-fache oder weniger beträgt, bezogen auf ein Gewicht von Molybdän, das in dem Ammoniummolybdat enthalten ist; und

ein Wismutkomponenten-Rohmaterial nur aus Wismutnitrat besteht, ein Gewicht von einer wässrigen Salpetersäurelösung zum Lösen das 2,3-fache oder mehr beträgt, bezogen auf ein Gewicht von Wismut, das in dem Wismutnitrat enthalten ist, und eine Salpetersäurekonzentration der wässrigen Salpetersäurelösung zum Lösen von Wismutnitrat darin 10 Gew.-% oder mehr beträgt.

3. Verfahren zur Herstellung eines ungesättigten Aldehyds und/oder einer ungesättigten Carbonsäure nach Anspruch 1, wobei der komplexe Metalloxidkatalysator ein sphärisch beschichteter Katalysator ist, bei dem katalytisch aktive Komponenten auf einer Oberfläche eines inerten Trägers getragen werden.

4. Verfahren zur Herstellung eines ungesättigten Aldehyds und/oder einer ungesättigten Carbonsäure nach einem der Ansprüche 1 oder 3,
wobei eine Ladung des Alkens in dem in den mehrröhrigen Reaktor zuzuführenden Rohmaterialgas das 100-fache oder mehr (in einem Standard-Zustand umgewandelt) bezogen auf ein Einheitskatalysatorvolumen pro Stunde beträgt.

5. Verfahren zur Herstellung eines ungesättigten Aldehyds und/oder einer ungesättigten Carbonsäure nach einem der Ansprüche 1 oder 3 bis 4,
wobei eine Ladung des Alkens in dem in den mehrröhrigen Reaktor zuzuführenden Rohmaterialgas das 150-fache oder mehr (in einem Standard-Zustand umgewandelt) bezogen auf ein Einheitskatalysatorvolumen pro Stunde beträgt.

6. Verfahren zur Herstellung eines ungesättigten Aldehyds und/oder einer ungesättigten Carbonsäure nach einem der Ansprüche 1 oder 3 bis 5,
wobei eine Konzentration des Alkens, das in dem in den mehrröhrigen Reaktor zuzuführenden Rohmaterialgas enthalten ist, 8,5 Vol.-% oder weniger beträgt.

7. Verfahren zur Herstellung eines ungesättigten Aldehyds und/oder einer ungesättigten Carbonsäure nach einem der Ansprüche 1 oder 3 bis 6.
wobei alle der im mehrröhrigen Reaktor gefüllten Katalysatorschichten nicht durch physikalisches Mischen des komplexen Metalloxidkatalysators und einer inerten Substanz verdünnt werden.

8. Verfahren zur Herstellung von Acrolein und/oder Acrylsäure oder Methacrolein und/oder Methacrylsäure durch das Verfahren zur Herstellung eines ungesättigten Aldehyds und/oder einer ungesättigten Carbonsäure nach einem der Ansprüche 1 oder 3 bis 7.

## Revendications

1. Procédé de production d'un aldéhyde insaturé et/ou d'un acide carboxylique insaturé, le procédé comprenant la soumission d'un alcène à une oxydation partielle catalytique en phase gazeuse avec de l'oxygène moléculaire en utilisant un réacteur multitubulaire ayant un catalyseur d'oxyde métallique complexe chargé dans celui-ci, obtenant ainsi un aldéhyde insaturé et/ou un acide carboxylique insaturé correspondant, dans lequel
deux couches de catalyseur ou plus contenant chacune un catalyseur d'oxyde métallique complexe sont empilées dans une direction axiale du tube, réalisant ainsi un chargement multicouche ;
une formulation du catalyseur d'oxyde métallique complexe contenu dans une couche de catalyseur est différente d'une formulation du catalyseur d'oxyde métallique complexe contenu dans au moins l'une des autres couches de catalyseur;
un rapport d'une quantité de composant de bismuth à une quantité de composant de molybdène de la couche de catalyseur du côté le plus proche de l'entrée de gaz dans l'axe du tube est supérieur à un rapport d'une quantité de composant de bismuth à une quantité de composant de molybdène de la couche de catalyseur du côté le plus proche de la sortie de gaz dans l'axe du tube ;
dans la totalité des deux couches de catalyseur adjacentes l'une à l'autre, un rapport d'une quantité de composant de bismuth à une quantité de composant de molybdène de la couche de catalyseur du côté de l'entrée de gaz dans l'axe du tube est supérieur ou égal à un rapport d'une quantité de composant de bismuth à une quantité de composant de molybdène de la couche de catalyseur du côté de la sortie de gaz dans l'axe du tube ; et
la couche de catalyseur du côté le plus proche de la sortie de gaz dans l'axe du tube contient un catalyseur contenant un composé représenté par la formule (1) suivante :

$$Mo_{12}Bi_aFe_bCo_cNi_dX_eY_fZ_gO_h \qquad (1)$$

dans lequel
X est au moins un élément choisi dans le groupe constitué du magnésium (Mg), du calcium (Ca), du manganèse (Mn), du cuivre (Cu), du zinc (Zn), du cérium (Ce) et du samarium (Sm) ; Y est au moins un élément choisi dans le groupe constitué du bore (B), du phosphore (P), de l'arsenic (As), de l'antimoine (Sb), du tungstène (W), du silicium (Si) et de l'aluminium (Al) ; Z est au moins un élément choisi dans le groupe constitué du sodium (Na), du potassium (K), du rubidium (Rb) et du césium (Cs) ; a à h représentent les rapports atomiques des composants respectifs ; a = 0,40 ou plus et moins de 0,80 ; b = 1,0 à 2,5 ; c = 3,0 à 7,5 ; d = 2,0 à 3,5 ; e = 0 à 10 ; f = 0 à 10 ; g = 0,01 à 1,0; h est exprimé par une valeur numérique satisfaisant les états d'oxydation d'autres éléments ; d/a est supérieur à 2,5 et de 8,8 ou moins ; d/g est de 2,0 ou plus et de 350 ou moins ; et a/g est de 0,4 ou plus et de moins de 80.

2. Procédé de production du composé représenté par la formule (1) selon la revendication 1,
dans lequel dans une étape de préparation du composé représenté par la formule (1), une matière première de composant de molybdène est uniquement constituée d'un molybdate d'ammonium, et un poids d'eau pour la dissolution est de 8,5 fois ou moins par rapport à un poids de molybdène contenu dans le molybdate d'ammonium ; et une matière première de composant de bismuth est uniquement constituée de nitrate de bismuth, un poids d'une solution aqueuse d'acide nitrique pour la dissolution est de 2,3 fois ou plus par rapport à un poids de bismuth contenu dans le nitrate de bismuth, et une concentration d'acide nitrique de la solution aqueuse d'acide nitrique pour la dissolution de nitrate de bismuth dans celle-ci est de 10 % en poids ou plus.

3. Procédé de production d'un aldéhyde insaturé et/ou d'un acide carboxylique insaturé selon la revendication 1,
dans lequel le catalyseur d'oxyde métallique complexe est un catalyseur revêtu sphérique dans lequel les composants catalytiques actifs sont supportés sur une surface d'un vecteur inerte.

4. Procédé de production d'un aldéhyde insaturé et/ou d'un acide carboxylique insaturé selon l'une quelconque des revendications 1 ou 3,
dans lequel une charge de l'alcène dans la matière première gazeuse devant être introduite dans le réacteur multitubulaire est de 100 fois ou plus (convertie dans un état standard) par rapport à un volume unitaire de catalyseur par heure.

5. Procédé de production d'un aldéhyde insaturé et/ou d'un acide carboxylique insaturé selon l'une quelconque des revendications 1 ou 3 à 4,
dans lequel une charge de l'alcène dans la matière première gazeuse devant être introduite dans le réacteur multitubulaire est de 150 fois ou plus (convertie dans un état standard) par rapport à un volume unitaire de catalyseur par heure.

6. Procédé de production d'un aldéhyde insaturé et/ou d'un acide carboxylique insaturé selon l'une quelconque des revendications 1 ou 3 à 5,
dans lequel une concentration de l'alcène contenu dans la matière première gazeuse devant être introduite dans le réacteur multitubulaire est de 8,5 % en volume ou moins.

7. Procédé de production d'un aldéhyde insaturé et/ou d'un acide carboxylique insaturé selon l'une quelconque des revendications 1 ou 3 à 6,
dans lequel toutes les couches de catalyseur chargées dans le réacteur multitubulaire ne sont pas diluées par mélange physique du catalyseur d'oxyde métallique complexe et d'une substance inerte.

8. Procédé de production d'acroléine et/ou d'acide acrylique, ou de méthacroléine et/ou d'acide méthacrylique, par le procédé de production d'un aldéhyde insaturé et/ou d'un acide carboxylique insaturé selon l'une quelconque des revendications 1 ou 3 à 7.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001226302 A
- JP H6192144 A
- JP 2007511565 T
- JP 2001048817 A
- WO 2015008815 A
- JP 2015138375 A